# EUROPÄISCHE PATENTSCHRIFT

(11) **EP 2 060 251 B1**
(45) Veröffentlichungstag und Bekanntmachung des Hinweises auf die Patenterteilung: **23.03.2011**
(21) Anmeldenummer: 07022346.6
(22) Anmeldetag: 17.11.2007
(51) Int. Cl.: A61K 8/97, A61K 36/00, C11B 1/10, B01F 17/00, A61K 8/06

(54) **Verfahren zur Herstellung von Wirkstoffkonzentraten**
Method for manufacturing active agent concentrates
Procédé de fabrication de concentrés de matière active

(43) Veröffentlichungstag der Anmeldung: 20.05.2009
(73) Patentinhaber: Cognis IP Management GmbH, 40589 Düsseldorf (DE)
(72) Erfinder: Beverungen, Carsten, 40591 Düsseldorf (DE); Weiss, Albrecht, 40764 Langenfeld (DE); Mahnke, Eike, Ulf, 42553 Velbert (DE); Gutsche, Bernard, 40724 Hilden (DE)
(74) Vertreter: Fabry, Bernd

(56) Entgegenhaltungen:
- WO-A-2006/087119
- DATABASE WPI Week 199338 Thomson Scientific, London, GB; AN 1993-301490 XP002488993 & SU 1 763 475 A1 (AS UZB HEAT PHYS) 23. September 1992 (1992-09-23)

## Beschreibung

### Gebiet der Erfindung

Die Erfindung befindet sich auf dem Gebiet der pflanzlichen Wirkstoffe und betrifft die Verwendung von PIT-Emulsionen als Extraktionsmittel.

### Stand der Technik

Seit längerer Zeit ist ein Trend zu beobachten, Wirkstoffe nicht synthetischen, sondern natürlichen Ursprungs sowohl für die Herstellung von kosmetischen und pharmazeutischen Zubereitungen, als auch für Nahrungsmittel- und Nahrungsmittelzusatzstoffe einzusetzen. Die Gründe dafür sind sehr unterschiedlich: zum einen wird vom Verbraucher der Hinweis auf einen Wirkstoff, der direkt der Natur entnommen und nicht im Labor synthetisiert worden ist, auch mit höheren Preisen honoriert, zum anderen sind die Verfahren zur Gewinnung dieser Wirkstoffe aus vorwiegend pflanzlichen Quellen so stark verbessert worden, dass die so erhaltenen Wirkstoffkonzentrate auch unter ökonomisch sinnvollen Bedingungen hergestellt werden können. Abgesehen davon, lassen sich viele Wirkstoffe immer noch leichter in einer Zelle als in einem Reaktor herstellen, wenn denn überhaupt eine technische Syntheseroute bekannt ist.

Unter der Bezeichnung "natürlicher Rohstoff" ist indes immer noch ein Produkt zu verstehen, welches auf chemischem Wege, in der Regel durch Extraktion gewonnen wird, bei diesem Verfahren jedoch keine strukturelle Veränderung erfährt. Die Herstellung solcher Extrakte erfolgt gewöhnlich durch wässrigen, alkoholischen oder wässrig-alkoholischen Auszug der Pflanzen bzw. Pflanzenteile bzw. der Blätter oder Früchte. Grundsätzlich sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss geeignet, wobei die Perkolation die Methode der Wahl für technische Umsetzungen ist. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, wie beispielsweise wässriges Methanol, Ethanol, Pentan, Hexan, Heptan, Aceton, Propylenglykolen, Polyethylenglykolen sowie Ethylacetat. Die Extraktion erfolgt in der Regel bei 20 bis 100 °C, bevorzugt bei 30 bis 90 °C, insbesondere bei 60 bis 80 °C.

Von Nachteil ist indes, dass die Extraktionsmittel jeweils an die Polarität der gewünschten Wirkstoffe angepasst werden müssen, was es aufwendig macht, an einem Tag jenen und am nächsten Tag einen anderen Wirkstoff in der gleichen Anlage herzustellen. Ebenfalls unbefriedigend ist, dass auch mit Hilfe von jeweils auf den Wirkstoff optimierten Lösemitteln in kurzen Zeiten nur vergleichsweise geringe Mengen extrahiert werden können oder umgekehrt für einen hohen Extraktionsgrad lange Extraktionszeiten erforderlich sind. Aus der SU 1763475 A1 ist dabei die Verwendung von O/W-Emulsionen zur Extraktion von pflanzlichen Ölen aus Pressrückständen bekannt.

Die Aufgabe der vorliegenden Erfindung hat demnach darin bestanden, neue Extraktionsmittel zur Verfügung zu stellen, mit denen die Nachteile des Stands der Technik überwunden werden können und mit deren Hilfe es insbesondere möglich ist, weitgehend unabhängig von der Natur der zu extrahierenden Wirkstoffe mit geringem technischen Aufwand in kurzen Zeiten höhere Extraktionsausbeuten zu erzielen.

### Beschreibung der Erfindung

Gegenstand der Erfindung ist ein Verfahren zur Herstellung von Wirkstoffkonzentraten welches sich dadurch auszeichnet, dass man ein wirkstoffhaltiges Ausgangsmaterial
(i)
   (a) entweder mit einer PIT-Emulsion in Kontakt bringt, die Mischungen auf eine Temperatur oberhalb der Phaseninversionstemperatur der PIT-Emulsion erhitzt und dort für einen ausreichenden Zeitraum belässt, oder
   (b) mit einer zur Bildung von PIT-Emulsionen geeigneten wässrigen Mischung aus Ölkörpern und Emulgatoren in Kontakt bringt, die Mischung unter starker Scherung auf eine Temperatur oberhalb der Phaseninversionstemperatur unter *in*-*situ* Ausbildung einer PIT-Emulsion erhitzt und dort für einen ausreichenden Zeitraum belässt,
(ii) die Mischungen abkühlt und filtriert sowie gegebenenfalls
(iii) einer Ultrafiltration und/oder
(iv) einer weiteren Lösemittelextraktion unterwirft.

Überraschenderweise wurde gefunden, dass die Extraktion von wirkstoffhaltigen Ausgangsmaterialien, speziell pflanzlichen Ausgangsstoffen wie beispielsweise Wurzeln, Blätter, Schalen, Stiele, Kerne, Saaten und dergleichen, mit Hilfe von PIT-Emulsionen oberhalb der Phaseninversionstemperatur in kürzeren Zeiten zu deutlich höheren Ausbeuten führt. Die Eignung der PIT-Emulsionen ist dabei in weiten Grenzen von der Natur und insbesondere von der Polarität der zu extrahierenden Wirkstoffe unabhängig, was deren technischen Einsatz erleichtert. Ein weiterer Vorteil besteht darin, dass man die PIT-Emulsionen entweder unmittelbar als Extraktionsmittel einsetzen kann oder aber deren Komponenten - Ölkörper, Emulgatoren, Wasser - den Ausgangsstoffen getrennt zusetzt und dann die PIT-Emulsion im Zuge der Extraktion *in*-situ herstellt.

### Wirkstoffe und deren Ausgangsmaterialien

Ohne die vorliegende Erfindung darauf einzugrenzen, stellen die bevorzugten Wirkstoffe aktive Prinzipien dar, die aus Blättern, Blüten, Früchten, Schalen, Stängeln, Wurzeln, Saaten oder Kernen unterschiedlichster Pflanzen gewonnen werden. Typische Beispiele sind Extrakte der folgenden Pflanzen: *Ginkgo biloba, Camellia sinensis, Oleacea europensis, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Melissa officinalis, Medicago sativa, Valeriana officinalis, Hoodia gordonii, Castanea sativa*, *Salix alba*, *Tagetes* sowie *Hapagophytum procumbens.* Im Folgenden wird kurz auf die Zusammensetzung und die wesentlichen aktiven Wirkstoffe in den Extrakten eingegangen.

### Ginkgo biloba

Die aktiven Wirkstoffe der Extrakte, die aus den Blättern des Ginkgobaumes (*Ginkgo biloba*) gewonnen werden, sind Flavonoidglycosides, welche unter anderem (Iso)Quercitinglycoside, Kaempferol, Kaempferol-3-rhamnoside, Isorhamnetin, Luteolinglycoside, Sitosterolglycoside und insbesondere hexacyclische Terpenlactone, die so genannten Ginkgolide A, B, C, J, M und Bilobalide enthalten.

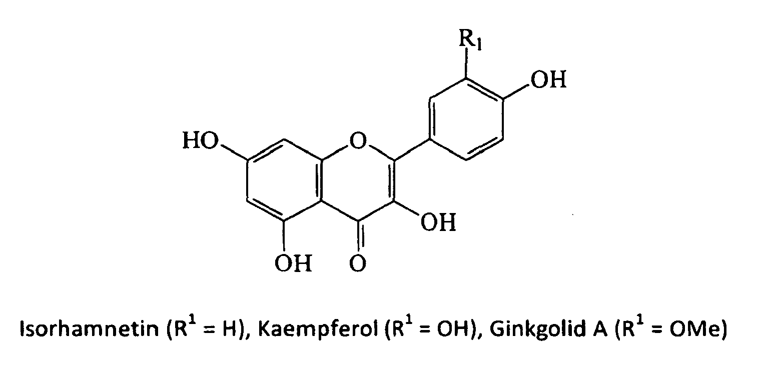

### Camellia sinensis

Die Blätter des Grünen Tees enthalten eine Vielzahl von Stoffen, wie z.B. Polysaccharide, flüchtige Öle, Vitamine, Mineralien, Purine und neben Alkaloiden, wie dem Koffein, insbesondere Polyphenole, bei denen es sich in der Regel um Catechine und Flavonoide handelt und die auch als "Tee-Tannine" bezeichnet werden.

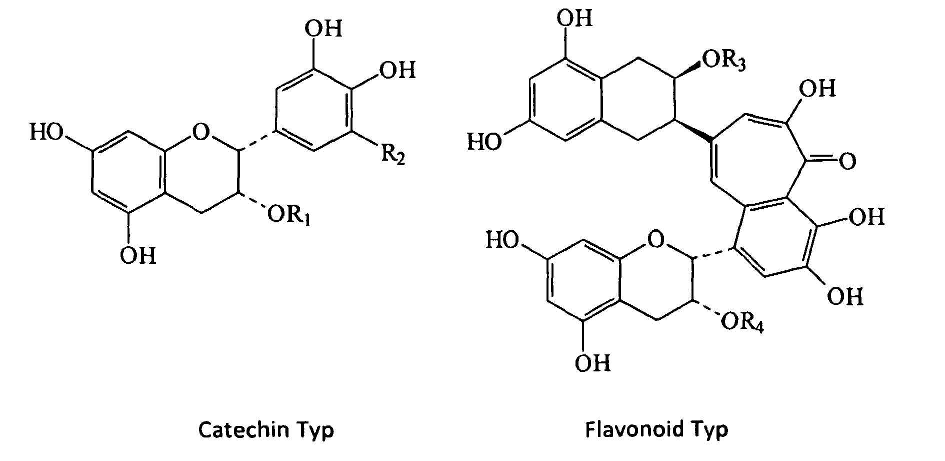

| **Komponenten** | **R1** | **R2** | **R3** | **R4** |
|---|---|---|---|---|
| (-)-Epicatechin | H | H | | |
| (-) Epigallocatechin | H | OH | | |
| (-) Epicatechin gallate | Galloyl | H | | |
| (-) Epigallocatechin gallate | Galloyl | OH | | |
| Theflavin | | | H | H |
| Theaflavin monogallate A | | | Galloyl | H |
| Theaflavin monogallat B | | | H | Galloyl |
| Theaflavin digallate | | | Galloyl | Galloyl |

### Oleacea europensis

Der Hauptbestandteil der Blätter des Olivenbaums (*Oleacea europensis*) ist das Antioxidants Oleuropein, das auch die wichtigste Quelle für Hydroxytyrosol darstellt.

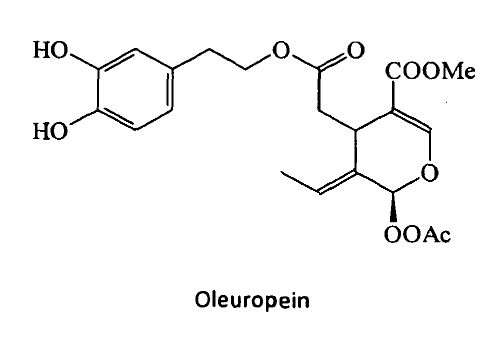

### Glyzyrrhiza glabra

Hauptbestandteil des Extraktes der Süßwurzel *Glyzyrrhiza glabra* ist die Glyzyrrhetinsäure.

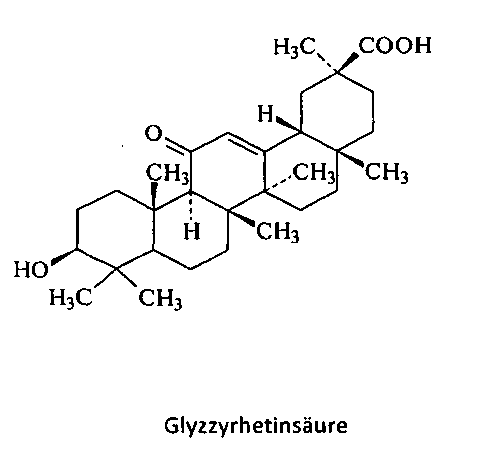

### Vaccinium myrtillus

Extrakte der gemeinen Blaubeere (*Vaccinium myrtillus*) enthalten eine Mischung von wenigstens 15 verschiedenen Anthocyanosides, wie beispielsweise dem folgenden:

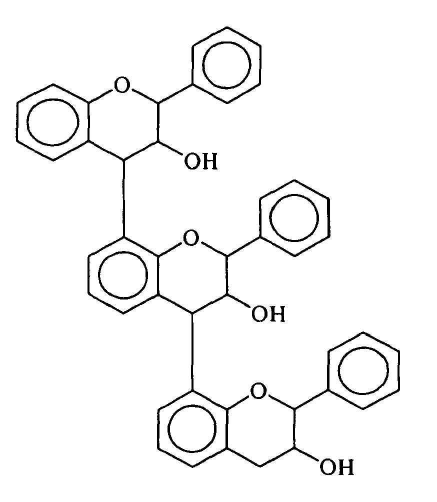

Üblicherweise, weisen die Extrakte 20 bis 25 Gew.-% Anthocyanoside, 5 bis 10 Gew.-% Tannine sowie geringe Mengen verschiedener Alkaloide, wie z.B. Myrtin und Epimyrtin, Phenolsäuren sowie Glycoside von Quercitrin, Isoquercitrin und Hyperosid auf.

### Trifolium pratense

Die Hauptbestandteile der Extrakte des Rotklees (*Trifolium pratense*) sind Isoflavone, wie z.B. Daidzein, Genestein, Formononentin and Biochanin A sowie deren Glucosides wie z.B. Ononin oder Sissostrin:

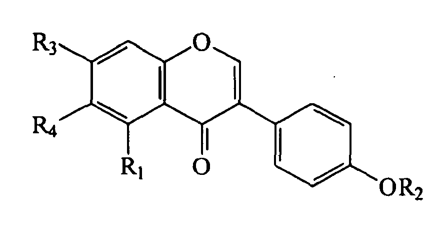

| **Isoflavonglucoside** | **R₁** | **R₂** | **R₃** | **R₄** |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

### Litchi sinensis

Extrakte, die aus den Schalen der Litchifrucht (*Litchi sinensis*) gewonnen werden, weisen hohe Gehalte an Flavonderivaten auf, wie z.B. 2-Phenyl-4H-1-benzopyranen, Flavanen, Flavan-3-olen (Catechinen, Catechinoligomeren), Flavan-3,4-diolen (Leucoanthocyaniden), Flavonen, Flavonolen und Flavononen. Der Hauptbestandteil wird jedoch ausgemacht von kondensierten Tanninen, sogenannten Procyanodolen (OPC). Diese Stoffe enthalten 2 bis 8 Monomere des Catechins oder eines Catechintyps, wie z.B. Procyanidin, Proanthocynidin, Procyanidole, Oligoprocyanidin, Leucoanthocyanidin, Leucodelphinin, Leucocyanin and Anthocyanogen. OPC, vorzugsweise Proanthocyanidin A2 (OPC A2) verhalten sich wie Vitamin P, vor allem mit Hinblick auf die Inhibierung von Matrixmetallproteinasen.

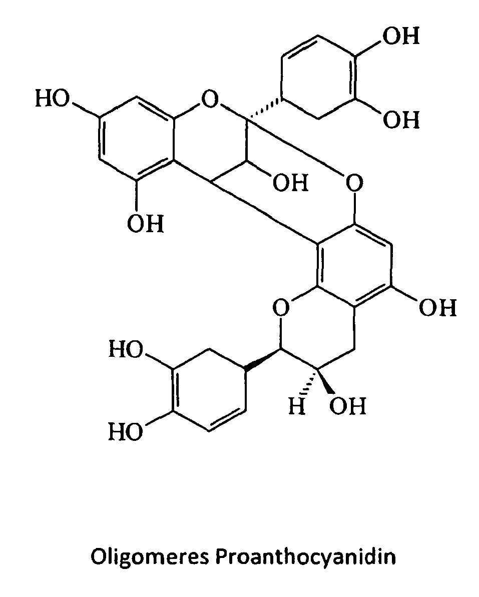

### Vitis vinifera

Die Hauptbestandteile aus Blättern, Wurzeln und insbesondere Schalen der Weintraube (*Vitis vinifera*)sind Polyphenole vom oben beschriebenen OPC-Typ.

### Brassica oleracea

Die Hauptbestandteile der Extrakte des Blumenkohls (*Brassica oleracea*) sind Aminosäuren, insbesondere Methionin und Cystein sowie die Glucosinolate, wie z.B. Glucoraphanin.

### Punica granatum

In den Extrakten des Granatapfels (*Punica granatum*) finden sich neben Zuckern und Zitronensäure insbesondere Delphinidin-1,2-glykoside sowie deren Aglykone.

### Petroselinium crispum

Hauptbestandteil des fetten Öls der Petersilie (*Petroselinium crispum*) ist die Petroselinsäure. Die Extrakte hingegen zeigen hohe Gehalte an Apiol (1-Allyl-2,5-dimethoxy-3,4-(methylendioxy)benzol,), sowie Apiin, Myristicin, Pinen und Selinen.

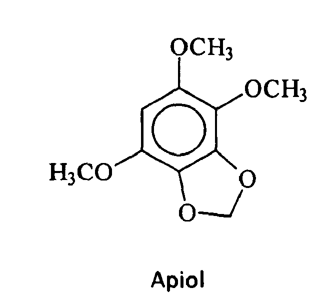

### Centella asiatica

Hauptbestandteile der Extrakte der *Centella asiatica* sind hochkondensierte Naphthensäuren, speziell Asiaticasäure, Madecassicasäure sowie deren Glycoside.

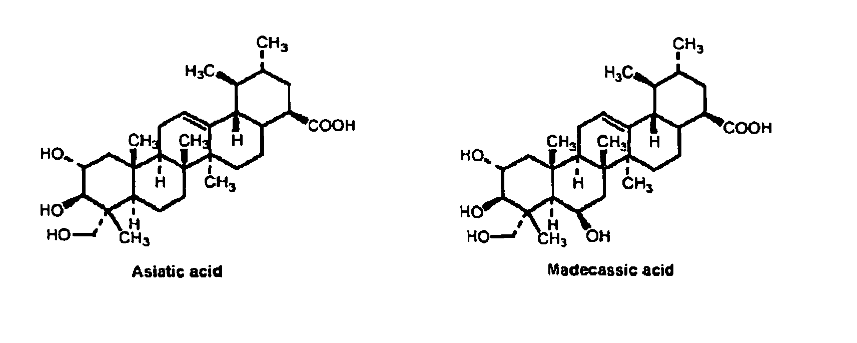

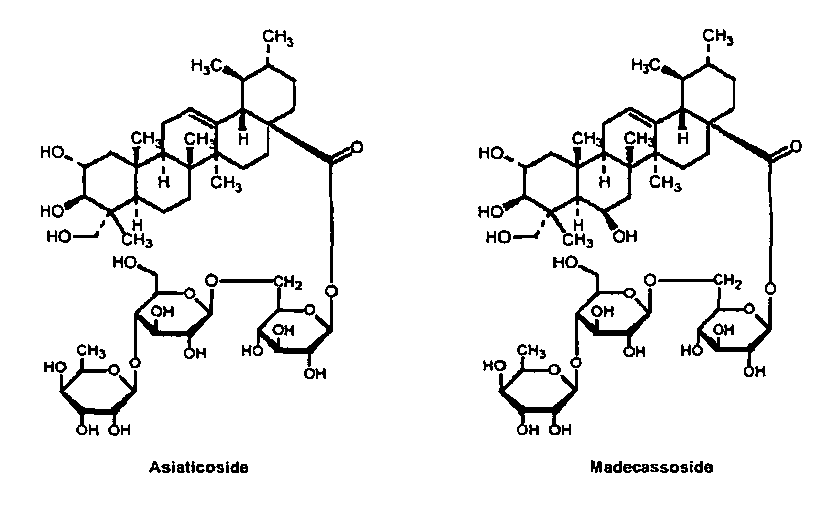

### Passiflora incarnata

Extrakte der Passionsfrucht (*Passiflora incarnata*) sind reich an Flavonen vom Typ des Apigenins und Luteolins sowie deren C-Glycoside.

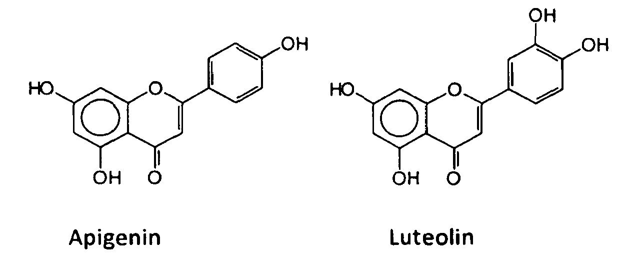

Des Weiteren enthalten sie 2"-B-D-Glucosides, Schaftoside and Isoschaftoside, Isovitexin, Isoorientin, Vicenin-2, Incenin-2, Daponanin sowie Spurenelement, nämlich vor allem Kalzium, Phosphor und Eisen.

### Medicago sativa

Extrakte der Alfalfa (*Medicago sativa*) sind reich an Isoflavonen, wie z.B. Daidzein, Genestein, Formononetin, Biochanin A und Tricin :

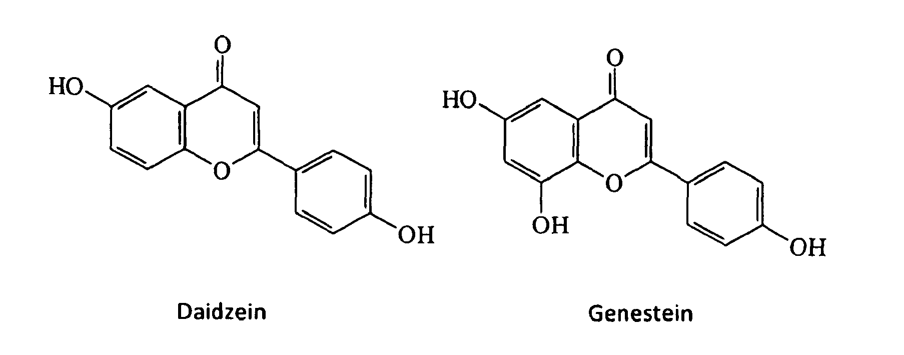

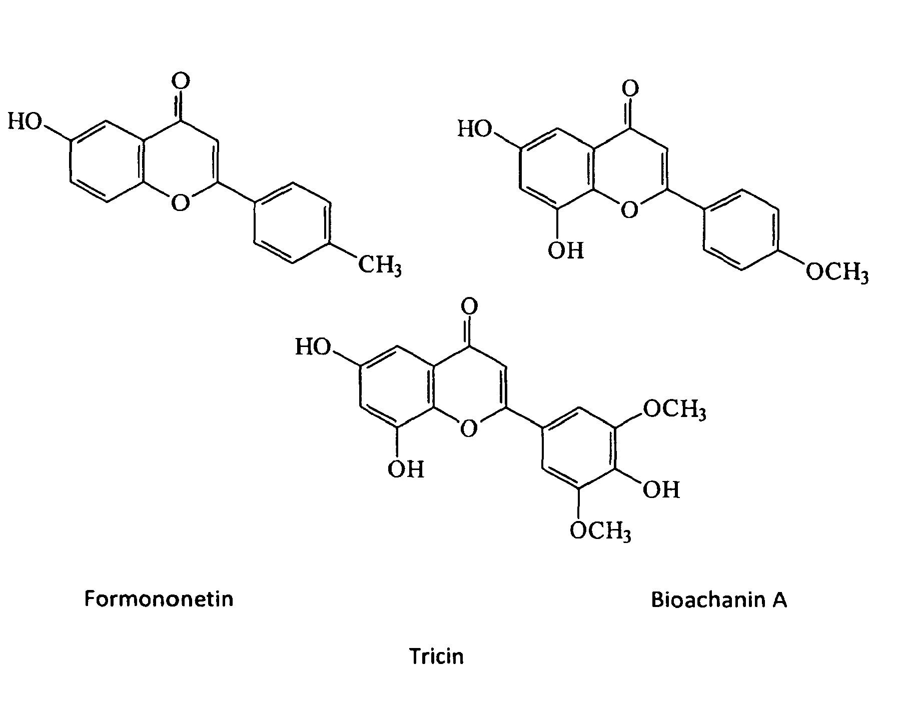

### Valeriana officinalis

Die Hauptbestandteile von Extrakten der *Valeriana officinalis* sind Valeriansäure, Valerianon sowie Borneolester.

### Castanea sativa

Rosskastanienextrakte (*Castanea sativa*) enthalten hauptsächlich Saponine sowie Escin, welches die Mischung zweier Glycoside darstellt, deren Aglycone sich von Proteoescigenin ableiten, während es sich bei den Zuckern entweder um Glucoronsäure oder zwei Molekülen D-Glucose handelt. Die beiden Glycoside unterscheiden sich in der Natur der Acylgruppen in der C22-Position.

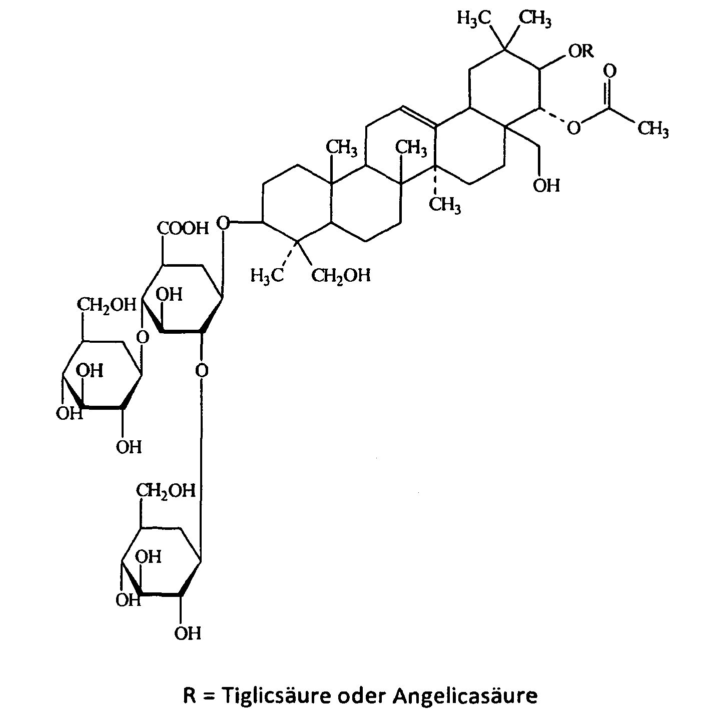

Während α-Escin ein amorphes Pulver darstellt, welches bei 225 bis 227 °C schmilzt und leicht wasserlöslich ist, liegt β-Escin (das auch als Flogencyl bezeichnet wird) in Form von Schuppen vor, die praktisch wasserunlöslich, aber leicht löslich in Alkohol sind.

### Hoodia gordonii

Hoodia, speziell *Hoodia gordonii*, ist eine Kaktuspflanze, die in Südafrika beheimatet und der einheimischen Bevölkerung seit langem als Mittel zur Bekämpfung des Hungergefühls bekannt ist. Es wird berichtet, dass in früheren Zeiten Buschmänner bei ihren Jagdzügen nur durch das Kauen von Hoodiawurzeln mehrere Wochen praktisch ohne Nahrung auskamen. In den vergangenen Jahren wurde gefunden, dass die erstaunlichen Eigenschaften dieser Pflanze mit ihrem hohen Gehalt an speziellen aktiven Steroidglykosiden zusammenhängen. In 2001/2002 gelang es erstmals, eine dieser Spezies zu isolieren und zu charakterisieren; sie wird in der Literatur seitdem als Substanz P57 bezeichnet:

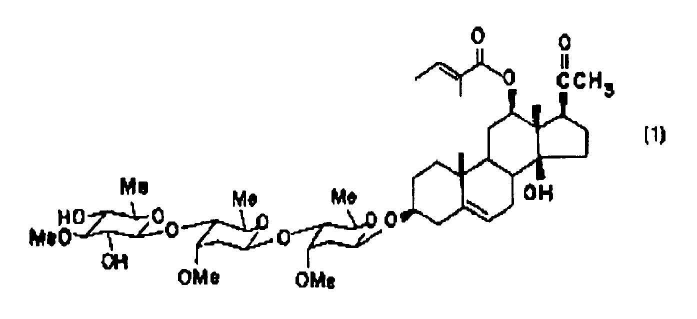

Aus der Patentliteratur ist bislang wenig über Hoodia und Hoodia-Extrakte bekannt. Aus der internationalen Patentanmeldung WO 98/046243 A1 (CSIR) sind jedoch pharmazeutische Zubereitungen auf Basis von Extrakten von Pflanzen des Genus *Trichocaulon* oder *Hoodia* bekannt, die über eine appetitzügelnde Wirkung verfügen sollen.

### Salix alba

Hauptbestandteile der Extrakte von *Salix alba* sind Phenolglykoside und insbesondere Salicylate wie z.B. Salicin, Salicortin und Tremulacin:

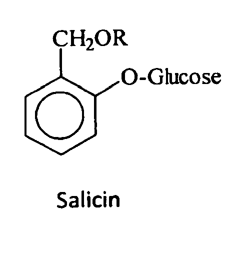

### Tagetes

Die unterschiedlichen Spezies, die zur Gattung Tagetes gehören, sind reich an Carotenoiden, insbesondere an Lutein und Zeaxanthin.

### Harpagophytum procumbens

Die Hauptbestandteile der Extrakte der Teufelskralle (*Harpagophytum procumbens*) sind Iridoidglucoside, Harpagoside, Harpagide und Procumbide.

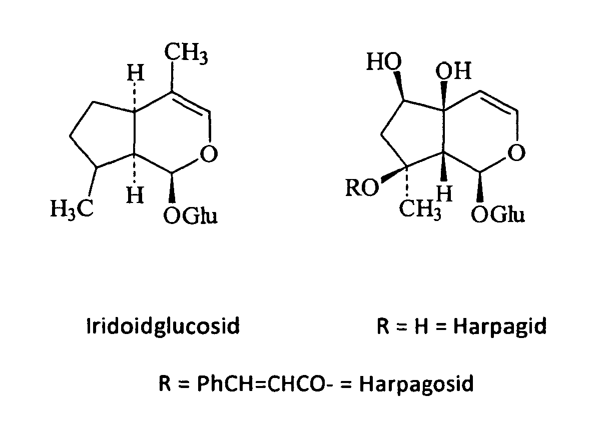

Des Weiteren findet man Stachylose und glycosylierte Phytosterole (z.B. β-Sitosterol), Flavonoide (z.B. Kaempferol, Luteolin), Phenolsäuren und glycosidische Phenylpropansäureestern (z.B. Verbacoside, Isoacteoside).

Andere Beispiele für geeignete Ausgangsstoffe, die der Extraktion mit PIT-Emulsionen unterworfen werden können, sind Schalen von Zitrusfrüchten oder pulverisierte und geröstete Kaffeebohnen.

### PIT-Emulsionen

Unter einer Emulsion versteht man ein fein verteiltes Gemisch zweier verschiedener (normalerweise nicht mischbarer) Flüssigkeiten ohne sichtbare Entmischung. *Hydrophile Flüssigkeiten* bilden hauptsächlich zwischenmolekularen Kräfte in Form von Wasserstoffbrücken aus. Bei *lipophilen Flüssigkeiten* bilden sich hingegen hauptsächlich zwischenmolekularen Van-der-Waals-Kräfte aus. Gibt man etwas Öl in Wasser, wird das Öl aufschwimmen. Zwischen Wasser und Öl hat sich eine *möglichst kleine* Grenzfläche gebildet. Zwischen den beiden Phasen können sich die oben genannten Kräfte nicht recht ausbilden. An der Grenzfläche bildet sich hingegen eine Grenzflächenspannung aus. Die Grenzflächenspannung ist der Antrieb eine *möglichst kleine* Grenzfläche zu bilden und verhindert damit die Existenz einer Emulsion. Zur Herstellung und Stabilisierung einer Emulsion sind daher spezielle grenzflächenaktive Substanzen mit emulgierenden Eigenschaften notwendig. Die Grenzflächenspannung an der Öl-Wasser-Phasengrenzfläche wird durch den Emulgator deutlich gesenkt, wodurch die Vermischung erst möglich wird. Emulsionen sind trotzdem instabile Systeme, haben also eine begrenzte Lebensdauer. Das so genannte "Brechen der Emulsion" erfolgt, da die Größe der Grenzflächen durch Zusammenfließen von Tröpfchen zu größeren Tröpfchen verringert wird. Eine PIT-Emulsion unterscheidet sich von anderen Emulsionen dadurch, dass sie sehr stabile Ölpartikel im µm-Bereich bilden und bei geeigneten Temperaturen mit Wasser verdünnbar sind. Sehr charakteristisch ist, dass das Öl:Emulgator-Verhältnis ein Mehrfaches der normalen Emulsionen beträgt, nämlich in der Regel bei 10 bis 20 Gew.-% Emulgator bezogen auf den Ölkörper. Die Herstellung solcher Systeme ist aus dem Stand der Technik hinreichend bekannt; stellvertretend hierzu sei auf die Veröffentlichungen von T. Förster et al. in Cosm. Toil. 106, 49-52 (1991**)** und Int. J. Cosm. Sci. 16, 84-92 (1994**)** hingewiesen.

### Ölkörper

Zur Herstellung der PIT-Emulsionen kommen als Ölkörper beispielsweise die folgenden Vertreter in Betracht: Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. Squalan, Squalen oder Dialkylcyclohexane.

### Emulgatoren

Als Emulgatoren kommen für die Herstellung der PIT-Emulsionen beispielsweise nichtionogene Tenside aus mindestens einer der folgenden Gruppen in Frage:
o Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
o Alkyl- und/oder Alkenyloligoglykoside mit 8 bis 22 Kohlenstoffatomen im Alk(en)ylrest und deren ethoxylierte Analoga;
o Anlagerungsprodukte von 1 bis 15 Mol Ethylenoxid an Ricinusöl und/oder gehärtetes Ricinusöl;
o Anlagerungsprodukte von 15 bis 60 Mol Ethylenoxid an Ricinusöl und/oder gehärtetes Ricinusöl;
o Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
o Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
o Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
o Mono-, Di- und Trialkylphosphate sowie Mono-, Di- und/oder Tri-PEG-alkylphosphate und deren Salze;
o Wollwachsalkohole;
o Polysiloxan-Polyalkyl-Polyether-Copolymere bzw. entsprechende Derivate;
o Block-Copolymere z.B. Polyethylenglycol-30 Dipolyhydroxystearate;
o Polymeremulgatoren, z.B. Pemulen-Typen (TR-1,TR-2) von Goodrich oder Cosmedia^{®} SP von Cognis;
o Polyalkylenglycole sowie
o Glycerincarbonat.

### Alkoxylate

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### Sorbitanester

Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina@), Polyglyceryl-4 Caprate (Polyglycerol Caprate T2010/90), Polyglyceryl-3 Cetyl Ether (Chimexane@ NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Zur Herstellung der PIT-Emulsionen werden die Ölkörper und die Emulgatoren wie weiter oben beschrieben üblicherweise in Gewichtsmengen im Bereich von 90:10 bis 70:30 und vorzugsweise um 80:20 eingesetzt und mit der 3 bis 10-fachen Menge Wasser in an sich bekannter Weise, d.h. unter Eintrag hoher Rührenergie zur Emulsion verarbeitet. Die auf diese Weise erhältlich Emulsionen weisen einen Tröpfchendurchmesser im Bereich von 0,01 bis 0,5 µm und vorzugsweise 0,03 bis 0,3 µm auf, wobei vorzugsweise wenigstens 50 % aller Tröpfchen einen Durchmesser von 0,05 bis 0,09 µm und insbesondere 0,04 bis 0,06 µm besitzen. Die Herstellung der PIT-Emulsionen kann entsprechend dem Verfahren des Stands der Technik diskontinuierlich, also im Batch-Betrieb erfolgen, vorzugsweise wird sie jedoch kontinuierlich durchgeführt, da auf diesem Wege eine engere Tröpfchengrößenverteilung erzielt wird. Solche Produkte sind zum einen natürlich für die technische Weiterverwendung nützlich, sie eignen sich aber auch beispielsweise für den Einsatz in Reihenuntersuchungen ("screening assays"), die üblicherweise auf wässrige Testlösungen ausgerichtet sind.

### Durchführung des Extraktionsverfahrens

In der ersten Ausführungsform der vorliegenden Erfindung wird zunächst die PIT-Emulsion nach einem der oben geschilderten Verfahren unter Einsatz einer Öl- und einer Wasserphase hergestellt, wobei vorzugsweise auf die kontinuierliche Mikroreaktionsherstellweise zurückgegriffen wird. In der Regel werden die wirkstoffhaltigen Ausgangsstoffe in den PIT-Emulsionen suspendiert und unter Erwärmen bis über die jeweilige Phaseninversionstemperatur erhitzt. Ist diese nicht bekannt, so kann die ?ildung der PIT-Emulsion über die Leitfähigkeit verfolgt werden: mit steigender Temperatur geht die Leitfähigkeit mit der Abnahme der Menge an wässriger kontinuierlicher Phase kontinuierlich zurück und erreicht oberhalb der PIT den Wert Null. Die Extraktion wird bei Erreichen bzw. Überschreiten der PIT über eine ausreichende Zeitspanne durchgeführt, die Einzelfall sehr unterschiedlich sein kann, jedoch in aller Regel wenigstens bei einer Stunde und selten mehr als 4 Stunden beträgt. Anschließend werden die Mischungen wieder abgekühlt und die unlöslichen Bestandteile abfiltriert. Die so erhaltenen Wirkstoffkonzentrate können bereits verkaufsfähige Endprodukte darstellen, in vielen Fällen empfiehlt sich jedoch eine weitere Aufreinigung und Anreicherung. Dazu gehört insbesondere eine erneute Auftrennung der PIT-Emulsion in eine wässrige und ölhaltige Phase, was beispielsweise dadurch erreicht wird, dass man die Emulsionen durch Ultrafiltration bzw. Membranfiltration bricht. Analytisch lässt sich nachweisen, dass - wirkstoffabhängig - der ganz überwiegende Teil, d.h. mehr als 80 und insbesondere mehr als 90 % der - in der Regel lipophilen - Wirkstoffe in der organischen Phase zu finden sind. Die organische Phase kann als solche nun ebenfalls als Verkaufsprodukt, beispielsweise für die kosmetische Industrie verwendet werden. Es ist jedoch ebenfalls möglich, eine weitere Lösemittelextraktion einzusetzen und die Wirkstoffe in einer polaren organischen Phase anzureichern, aus der sie dann durch Kristallisation oder Sprüh- oder Gefriertrocknung in fester Form gewonnen werden.

Alternativ lässt sich die Extraktion auch unter *in-situ* Bildung der PIT-Emulsionen durchführen. Anstelle der fertigen PIT-Emulsionen werden hierzu deren Einzelkomponenten eingesetzt und mit den wirkstoffhaltigen Ausgangsstoffen über die Phaseninversionstemperatur erhitzt. Dies erfolgt vorzugsweise unter Eintrag von Rührenergie, um möglichst kleine Tröpfchen zu erzeugen.

Der Vorteil dieser Verfahrensweise besteht darin, dass sich auch feuchtes Material für die Extraktion nutzen lässt, sich die Extraktionskomponenten nach betrieblichen Erfordernissen (z.B. Vermeidung von Explosionsschutz) auswählen lassen, durch die Wahl der Tensid/Ölmischung die Selektivität steuern lässt und die Art der Prozessführung das Einstellen einer tatsächlich minimalen Grenzflächenspannung möglich macht.

### Gewerbliche Anwendbarkeit

Ein weiterer Gegenstand der vorliegenden Patentanmeldung betrifft die Verwendung von PIT-Emulsionen als Extraktionsmittel für wirkstoffhaltige Ausgangsstoffe, insbesondere pflanzliche Materialien, speziell deren Blätter, Blüten, Früchte, Schalen, Stängel, Wurzeln, Saaten oder Kerne.

### Beispiele

### Beispiel 1

### Diskontinuierliche ("Batch") Herstellung einer PIT-Emulsion

In einem 250 ml-Kolben wurden 3,1 g Glycerinstearate (Cutina^{®} GMS), 6,9 g Ceteareth-12 (Eumulgin^{®} B1), 22,5 g Dicaprylylether (Cetiol^{®} OE), 22,5 g Dioctylcyclohexan (Cetiol^{®} S), 5 g Glycerin und 40 ml Wasser unter intensivem Rühren miteinander vermischt und innerhalb von 140 min von 25 °C auf 95 °C erwärmt. Anschließend wurde die Mischung innerhalb von 20 min wieder auf die Ausgangstemperatur abgekühlt. Um die Bildung der PIT-Emulsion zu verfolgen wurde die Leitfähigkeit als Funktion der Zeit verfolgt. Der Kurvenverlauf ist in Abbildung 1 wiedergegeben.

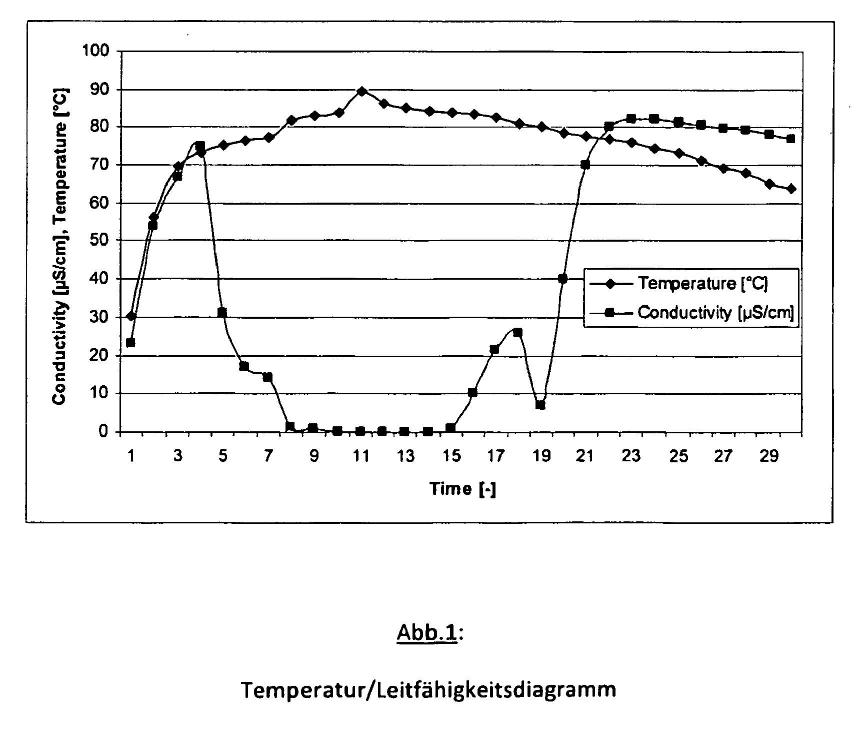

Die Leitfähigkeit nimmt mit steigender Temperatur ab und erreicht in der Umgebung der PIT-Temperatur den Wert Null; mit abnehmender Temperatur steigt die Leitfähigkeit dann wieder an. Dies ist damit zu erklären, dass unterhalb der PIT-Temperatur eine O/W-Emulsion vorliegt, bei der das Wasser die kontinuierliche Phase darstellt; oberhalb der PIT-Temperatur bildet dann das Öl die kontinuierliche Phase, was zum Verlust der Leitfähigkeit führt. Der Kurvenverlauf zeigt also deutlich die Bildung einer PIT-Emulsion an. Die so erhaltene Mischung zeigte die für PIT-Emulsionen bekannten Eigenschaften, nämlich hohe Stabilität, beliebige Mischbarkeit mit Wasser, sehr kleine Tröpfchengrößen sowie einen leichten bläulichen Schimmer. Zusätzlich wurden Proben entnommen und deren Tröpfchengröße mit einem Coulter LS Partikelgrößenmessgerät bestimmt (Abbildung 2)

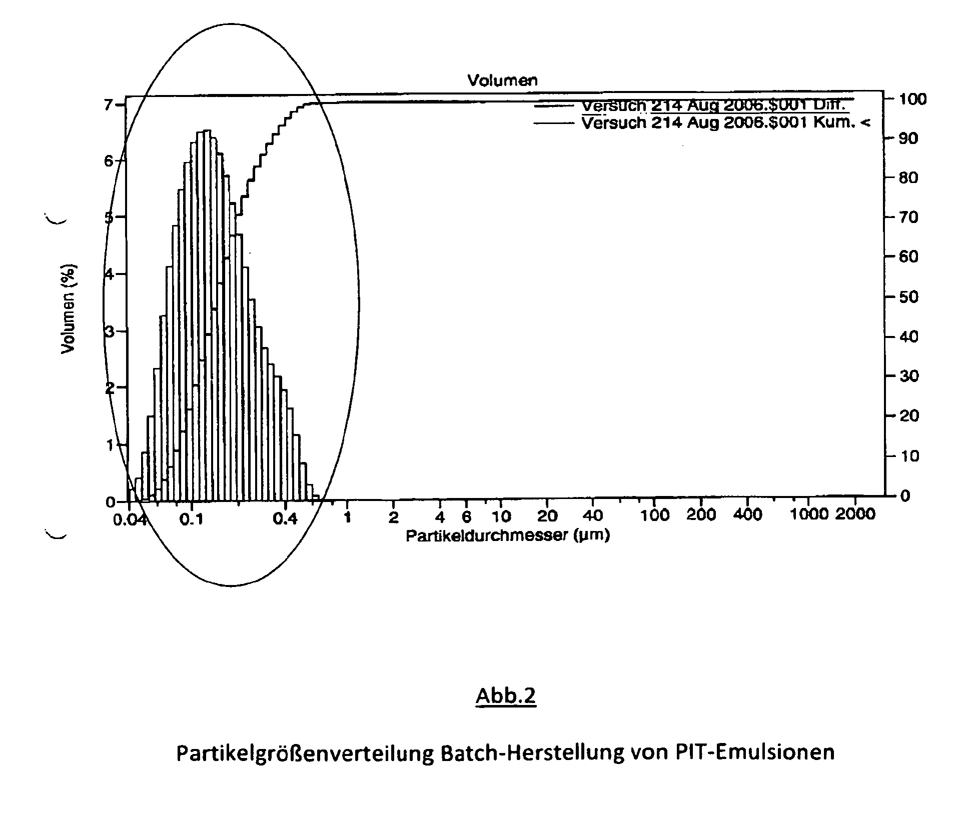

### Beispiel 2

### Kontinuierliche Herstellung einer PIT-Emulsion

Zur schnellen Temperierung und anschließenden schnellen Abkühlung bei der kontinuierlichen Herstellung der PIT-Emulsion wurde ein Mikromischer vom Typ Starlaminator 30, IMM mit zwei Mikrowärmetauschern zusammengeschaltet. Als Vorlagen für die Wasser- und Ölphase dienten zwei 15 L Doppelmantel-Glas-Reaktoren; das Fließschema ist in Abbildung 3 wiedergegeben.

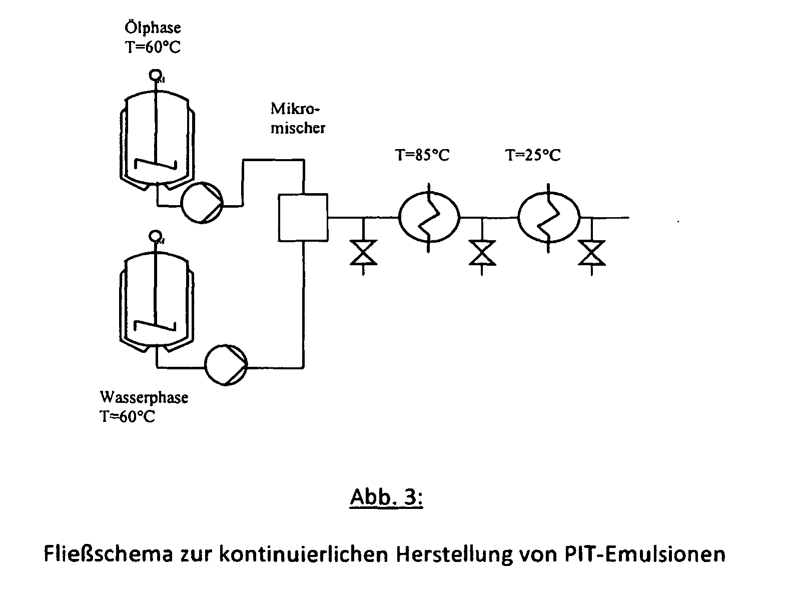

Die Ölphase bestehend aus 1,24 kg Cutina^{®} GMS, 2,76 kg Eumulgin^{®} B1, 9,00 kg Cetiol^{®} OE und 9,00 kg Cetiol^{®} S sowie die Wasserphase bestehend aus 2 kg Glycerin gelöst in 16 kg destilliertem Wasser wurden getrennt in einem Vorratsbehälter vorgelegt und auf T=60°C vorgewärmt. Beide Phasen wurden dabei gerührt. Die beiden Phasen (Ölphase: Wasserphase-1,22:1) wurden mit zwei unterschiedlichen Volumenströmen (6 L/h und 12 L/h) in den Mikromischer gepumpt und dort gemischt. Direkt nach dem StarLam30 Mischer wurde das Gemisch in einem Mikrowärmetauscher auf 85°C erhitzt, so dass die Phaseninversionstemperatur (81°C) überschritten wurde. Anschließend wurde die Emulsion in einem zweiten Mikrowärmeaustauscher auf eine Temperatur von T=25°C abgekühlt und in einem Vorratsgefäß gesammelt. Nach dem Mikromischer und nach den beiden Wärmetauschern wurde jeweils eine Probe entnommen und deren Temperatur und Leitfähigkeit bestimmt. Die Leitfähigkeit lag vor dem ersten Wärmetauscher bei ca. 55 µS/cm, sank nach dem ersten Mikrowärmetauscher auf einen Wert von 0,1 µS/cm um nach dem Abkühlen der Emulsion auf ca. 60 µS/cm anzusteigen. Die Bildung von echten PIT-Emulsionen konnte somit nachgewiesen werden.

Zusätzlich wurden Proben entnommen und deren Tröpfchengröße mit einem Coulter LS Partikelgrößenmessgerät bestimmt (Abbildung 4).

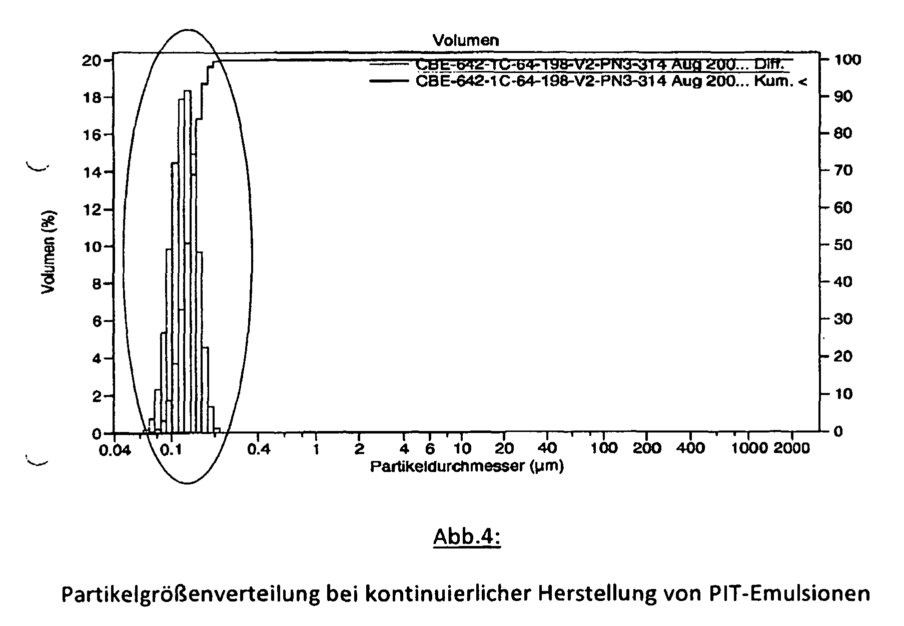

Die gemessenen Partikelgrößen sind mit ca. 0,125 µm kleiner als die Partikelgrößen, die im Batch-Verfahren erzielt werden (ca. 0,165 µm).

### Beispiel 3

### Extraktion von Pflanzenmaterial mit PIT-Emulsionen

Unterschiedliche pflanzliche Ausgangsstoffe (Olivenblätter, Orangenschalen, Kaffeepulver) wurde zerkleinert und zu einer Emulsion hergestellt nach den Beispielen 1 bzw. 2 gegeben. Die Zubereitungen wurden anschließend unter Rühren über 4 h auf eine Temperatur von ca. 90 °C, d.h. nahe an die Phaseninversionstemperatur erhitzt. Nach dem Abkühlen wurden die Mischungen zunächst filtriert, um unlösliche Rückstände abzutrennen, und dann einer Ultrafiltration unterworfen, um eine wässrige und eine ölhaltige Phase zu erzeugen. Der Gehalt an pflanzlichen Wirkstoffen in den beiden Phasen wurde durch Extraktion mit Acetonitril und nachfolgende HPLC-Analyse bestimmt. Die Ergebnisse sind in Tabelle 1 zusammengefasst:

**Tabelle 1**

| Extraktion von Wasser- und Ölphase | | | | |
|---|---|---|---|---|
| **Ausgangsstoff** | **Wasserphase [mg]** | **Ölphase [mg]** | **Gesamtmenge [mg]** | **Anteil Wirkstoffe in Ölphase [%]** |
| Olivenblätter | 60,0 | 340,0 | 400,0 | 85 |
| Orangenschalen | 46,0 | 114,0 | 160,0 | 91 |
| Kaffeepulver | 2,4 | 91,6 | 94,0 | 97 |

Auf diese Weise konnte gezeigt werden, dass die pflanzlichen Wirkstoffe ganz überwiegend, d.h. in Mengen zwischen 85 und 97 % in der Ölphase angereichert werden.

### Beispiel 4

### Vergleich PIT-Emulsionen mit anderen Extraktionsmitteln

1 kg Olivenblätter wurden mit jeweils 2 1 unterschiedlicher Extraktionsmittel 4 h bei 90 °C behandelt und anschließend der Gehalt an Oleuropein über HPLC bestimmt; dabei wurde der höchste Gehalt gleich 100 % gesetzt und alle anderen Ergebnisse darauf bezogen. Die Ergebnisse sind in Tabelle 2 zusammengefasst:

**Tabelle 2**

| Extraktion von Oleuropein | |
|---|---|
| **Extraktionsmittel** | **Oleuropeingehalt** |
| PIT-Emulsion gemäß Beispiel 2 | 100 |
| Ethanol | 74,3 |
| Acetonitril | 42,0 |
| Ethylmethylketon | 40,7 |
| Dicaprylylether | 0 |

Die Ergebnisse zeigen deutlich, dass unter Einsatz von PIT-Emulsionen etwa 25 % mehr Wirkstoffe extrahiert werden als beispielsweise mit dem Standardextraktionsmittel Ethanol.

### Beispiel 5

### In-situ Extraktion von Zeaxanthin und Lutein

2 kg einer Ölphase gemäß Beispiel 2 wurden bei T₁=50°C mit 750 g Tagetes-Blütenblättern für t=30 min gemischt. Anschließend wurde 5 1 destilliertes Wasser zugegeben und das Gemisch auf T₂=95°C erhitzt (ΔT=0,5°C/min). Nach dem Abkühlen wurden die Mischungen zunächst filtriert, um unlösliche Rückstände abzutrennen, und dann einer Ultrafiltration unterworfen, um eine wässrige und eine ölhaltige Phase zu erzeugen. Der Gehalt an Zeaxanthin und Lutein - bezogen auf die Gesamtmenge - betrug in der Ölphase 92 % und in der Wasserphase 8 %.

## Patentansprüche

1. Verfahren zur Herstellung von Wirkstoffkonzentraten, **dadurch gekennzeichnet, dass** man
(a) ein wirkstoffhaltiges Ausgangsmaterial mit einer PIT-Emulsion in Kontakt bringt,
(b) die Mischungen auf eine Temperatur oberhalb der Phaseninversionstemperatur der PIT-Emulsion erhitzt und dort für einen ausreichenden Zeitraum belässt,
(c) die Mischungen abkühlt und filtriert sowie gegebenenfalls
(d) einer Ultrafiltration und/oder
(e) einer weiteren Lösemittelextraktion unterwirft.

2. Verfahren zur Herstellung von Wirkstoffkonzentraten, **dadurch gekennzeichnet, dass** man
(a) ein wirkstoffhaltiges Ausgangsmaterial mit einer zur Bildung von PIT-Emulsionen geeigneten wässrigen Mischung aus Ölkörpern und Emulgatoren in Kontakt bringt,
(b) die Mischung unter starker Scherung auf eine Temperatur oberhalb der Phaseninversionstemperatur unter *in*-*situ* Ausbildung einer PIT-Emulsion erhitzt und dort für einen ausreichenden Zeitraum belässt,
(c) die Mischungen abkühlt und filtriert sowie gegebenenfalls
(d) einer Ultrafiltration und/oder
(e) einer weiteren Lösemittelextraktion unterwirft.

3. Verfahren nach den Ansprüchen 1 und/oder 2, **dadurch gekennzeichnet**, das man als wirkstoffhaltige Ausgangsmaterialien Blätter, Blüten, Früchte, Schalen, Stängel, Wurzeln, Saaten oder Kerne von Pflanzen einsetzt, die ausgewählt sind aus der Gruppe, die gebildet wird von *Ginkgo biloba, Camellia sinensis, Oleacea europensis, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Melissa officinalis, Medicago sativa, Valeriana officinalis, Hoodia gordonii, Castanea sativa, Salix alba, Tagetes* sowie *Hapagophytum procumbens.*

4. Verfahren nach mindestens einem der Ansprüche 1 bis 3, **dadurch gekennzeichnet, dass** man als wirkstoffhaltige Ausgangsmaterialien Schalen von Zitrusfrüchten oder Kaffeepulver einsetzt.

5. Verfahren nach mindestens einem der Ansprüche 1 bis 4, **dadurch gekennzeichnet, dass** man PIT-Emulsionen einsetzt, die Ölkörper und Emulgatoren im Gewichtsverhältnis 90:10 bis 70:30 enthalten.

6. Verfahren nach mindestens einem der Ansprüche 1 bis 5, **dadurch gekennzeichnet, dass** man PIT-Emulsionen einsetzt bzw. in-situ herstellt, die eine Tröpfchengrößenverteilung im Bereich von 0,01 bis 0,5 µm aufweisen.

7. Verfahren nach mindestens einem der Ansprüche 1 bis 6, **dadurch gekennzeichnet**, das man PIT-Emulsionen einsetzt bzw. in-situ herstellt, die eine Tröpfchengrößenverteilung aufweisen, bei denen mindestens 50 % aller Tröpfchen einen Durchmesser im Bereich von 0,05 bis 0,09 µm besitzen.

8. Verwendung von PIT-Emulsionen als Extraktionsmittel für wirkstoffhaltige Ausgangsstoffe.

9. Verwendung nach Anspruch 8, **dadurch gekennzeichnet, dass** die wirkstoffhaltigen Ausgangsstoffe Blätter, Blüten, Früchte, Schalen, Stängel, Wurzeln, Saaten oder Kerne von Pflanzen darstellen.

## Claims

1. A process for the preparation of active substance concentrates, wherein
(a) an active-substance-containing starting material is brought into contact with a PIT emulsion,
(b) the mixtures are heated to a temperature above the phase inversion temperature of the PIT emulsion, where they are held for a sufficient period of time,
(c) the mixtures are cooled and filtered and, if appropriate,
(d) subjected to ultrafiltration and/or
(e) a further solvent extraction.

2. A process for the preparation of active substance concentrates, wherein
(a) an active-substance-containing starting material is brought into contact with an aqueous mixture of lipids and emulsifiers which is suitable for forming PIT emulsions,
(b) the mixture is heated with strong shearing to a temperature above the phase inversion temperature with *in-situ* formation of a PIT emulsion, where the mixture is held for a sufficient period of time,
(c) the mixtures are cooled and filtered and, if appropriate,
(d) subjected to ultrafiltration and/or
(e) a further solvent extraction.

3. The process as claimed in claims 1 and/or 2, wherein the active-substance-containing starting materials employed are leaves, flowers, fruits, peels, stems, roots, seeds or pips of plants which are selected from the group formed by *Gingko biloba, Camellia sinensis, Oleacea europensis, Glyzyrrhiza glabra, Vaccinium myrtillus, Trifolium pratense, Litchi sinensis, Vitis vinifera, Brassica oleracea, Punica granatum, Petroselinum crispum, Centella asiatica, Passiflora incarnata, Melissa officinalis, Medicago sativa, Valeriana officinalis, Hoodia gordonii, Castanea sativa, Salix alba, Tagetes* and *Hapagophytum procumbens.*

4. The process as claimed in at least one of claims 1 to 3, wherein the active-substance-containing starting materials used are peels of citrus fruit or coffee powder.

5. The process as claimed in at least one of claims 1 to 4, wherein PIT emulsions are employed which comprise lipids and emulsifiers in a weight ratio of from 90:10 to 70:30.

6. The process as claimed in at least one of claims 1 to 5, wherein PIT emulsions are employed or prepared *in-situ* which have a droplet size distribution in the range of from 0.01 to 0.5 µm.

7. The process as claimed in at least one of claims 1 to 6, wherein PIT emulsions are employed or prepared *in-situ* which have a droplet size distribution where at least 50% of all droplets have a diameter in the range of from 0.05 to 0.09 µm.

8. The use of PIT emulsions as extractants for active-substance-containing starting materials.

9. The use according to claim 8, wherein the active-substance-containing starting materials are leaves, flowers, fruits, peels, stems, roots, seeds or pips of plants.

## Revendications

1. Procédé pour la préparation de concentrats de substances actives, **caractérisé en ce qu'**on (a) met un matériau de départ contenant des substances actives en contact avec une émulsion PIT,
(b) chauffe les mélanges à une température supérieure à la température d'inversion des phases de l'émulsion PIT et les laisse à cette température pendant un laps de temps suffisant,
(c) refroidit les mélanges et les filtre, ainsi que le cas échéant
(d) les soumet à une ultrafiltration et/ou
(e) à une autre extraction par un solvant.

2. Procédé pour la préparation de concentrats de substances actives, **caractérisé en ce qu'**on (a) met un matériau de départ contenant des substances actives en contact avec un mélange aqueux de corps huileux et d'émulsifiants approprié pour la formation d'émulsions PIT,
(b) chauffe le mélange sous fort cisaillement à une température supérieure à la température d'inversion des phases avec formation in situ d'une émulsion PIT et le laisse à cette température pendant un laps de temps suffisant,
(c) refroidit les mélanges et les filtre, ainsi que le cas échéant
(d) les soumet à une ultrafiltration et/ou
(e) à une autre extraction par un solvant.

3. Procédé selon les revendications 1 et/ou 2, **caractérisé en ce qu'**on utilise comme matériaux de départ contenant des substances actives des feuilles, des fleurs, des fruits, des peaux, des tiges, des racines, des graines ou des noyaux de plantes, qui sont choisies dans le groupe formé par les plantes *Ginkgo biloba, Camellia sinensis, Oleacea europensis, Glyzyrrhiza glabra, Vaccinium myrtillus, Trifolium pratense, Litchi sinensis, Vitis vinifera, Brassica oleracea, Punica granatum, Petroselinium crispum, Centella asiatica, Passiflora incarnata, Melissa officinalis, Medicago sativa, Valeriano officinalis, Hoodia gordonii, Castanea sativa, Salix alba, Tagetes* ainsi que *Hapagophytum procumbens.*

4. Procédé selon au moins l'une quelconque des revendications 1 à 3, **caractérisé en ce qu'**on utilise comme matériaux de départ contenant des substances actives des peaux d'agrumes ou de la poudre de café.

5. Procédé selon au moins l'une quelconque des revendications 1 à 4, **caractérisé en ce qu'**on utilise des émulsions PIT qui contiennent des corps huileux et des émulsifiants dans un rapport pondéral de 90:10 à 70:30.

6. Procédé selon au moins l'une quelconque des revendications 1 à 5, **caractérisé en ce qu'**on utilise ou prépare in situ des émulsions PIT qui présentent une répartition des grosseurs des gouttes dans la plage de 0,01 à 0,5 µm.

7. Procédé selon au moins l'une quelconque des revendications 1 à 6, **caractérisé en ce qu'**on utilise ou prépare in situ des émulsions PIT qui présentent une répartition des grosseurs des gouttes dans laquelle au moins 50 % de toutes les gouttes présentent un diamètre dans la plage de 0,05 à 0,09 µm.

8. Utilisation d'émulsions PIT comme agent d'extraction pour des substances de départ contenant des substances actives.

9. Utilisation selon la revendication 8, **caractérisée en ce que** les substances de départ contenant des substances actives sont des feuilles, des fleurs, des fruits, des peaux, des tiges, des racines, des graines ou des noyaux de plantes.
